# EUROPEAN PATENT SPECIFICATION

(11) **EP 3 281 996 B1**
(45) Date of publication and mention of the grant of the patent: **16.06.2021**
(21) Application number: 17185482.1
(22) Date of filing: 09.08.2017
(51) Int. Cl.: C09J 7/25, C08K 5/103, A61F 13/02, C09J 7/38

(54) **ADHESIVE SKIN PATCH MATERIAL AND ROLL BODY OF ADHESIVE SKIN PATCH MATERIAL**
KLEBENDES HAUTPFLASTERMATERIAL UND WALZENKÖRPER AUS KLEBENDEM HAUTPFLASTERMATERIAL
MATÉRIAU DE TIMBRE CUTANÉ ADHÉSIF ET CORPS DE ROULEAU DE MATÉRIAU DE TIMBRE CUTANÉ ADHÉSIF

(30) Priority: 09.08.2016 JP 2016156971
(43) Date of publication of application: 14.02.2018
(73) Proprietor: Nitto Denko Corporation, Ibaraki-shi, Osaka (JP)
(72) Inventor: SAKUMA, Takeshi, Ibaraki-shi, Osaka (JP); SUZUKI, Mai, Ibaraki-shi, Osaka (JP); NAKAMURA, Tetsuya, Ibaraki-shi, Osaka (JP)
(74) Representative: Grünecker Patent- und Rechtsanwälte PartG mbB

(56) References cited:
- EP-A1- 2 193 769
- EP-A1- 2 921 540
- EP-A2- 1 184 039

## Description

### FIELD OF THE INVENTION

The present invention relates to an adhesive skin patch material which is used in a state of being stuck on skin, and to a roll body of an adhesive skin patch material.

### BACKGROUND OF THE INVENTION

An adhesive skin patch material which has a base film and a pressure-sensitive adhesive layer provided on one side of the base film is used in a state of being affixed to a skin surface via the pressure-sensitive adhesive layer. When such an adhesive skin patch material is affixed to a skin surface, there may be cases where the skin surface gets sweaty and experiences itching, and even suffers irritation. Therefore high moisture permeability is required of adhesive skin patch materials.

Further, because the skin has an irregular and complex surface profile, the pressure-sensitive adhesive layer as a constituent member of an adhesive skin patch material is difficult to bring into absolute contact with the skin surface, but if the adhesive skin patch material is strongly bonded and fixed to a skin surface, it will probably cause skin stimulation at the time when peeled away from the skin surface. Accordingly, it has been desired to develop adhesive skin patch materials which have an excellent balance between the property of giving a good sense of fitness for skin without creating a perception of affixation while keeping good stickability on skin under their affixation to the skin and the property of reducing irritation to skin at the time when peeled away from the skin.
Patent Document 1: JP-A-H 1 0- 33655;
Patent Document 2: EP 2 921 540 A1;
Patent Document 3: EP 1 184 039 A2;
Patent Document 4 : EP 2 193 769 A1.

### SUMMARY OF THE IVENTION

In light of the foregoing status quo, the invention aims to provide an adhesive skin patch material which can ensure a good stickability on and a good sense of fitness for a skin surface and allows reductions in sweaty and itchy sensations under the affixation to the skin surface, and besides which can relieve a physical irritation to the skin surface when peeled away from the skin surface, and further aims to provide a roll body of such an adhesive skin patch material.

The present inventors have intensively studied over and over again in order to achieve the foregoing aims, and have found that the foregoing problems can be solved by designing an adhesive skin patch material so that the thickness of a polyurethane elastomer film as a base film is adjusted to fall within a specified range and an ingredient in a liquid or pasty state at room temperature is incorporated into a pressure-sensitive adhesive layer, thereby completing the invention. More specifically, the invention relates to an adhesive skin patch material which includes a polyurethane elastomer film, a pressure-sensitive adhesive layer provided on one side of the polyurethane elastomer film, and a release liner releasably laminated so as to cover the sticky surface of the pressure-sensitive adhesive layer, in which the polyurethane elastomer film has a thickness of 1 µm to 9 µm, wherein the pressure-sensitive adhesive layer has a thickness of 20 to 35 µm, and the pressure-sensitive adhesive layer contains a pressure-sensitive adhesive including an acrylic-based copolymer and an ingredient in a liquid or pasty state at room temperature.

The present adhesive skin patch material is an adhesive skin patch material which not only ensures a good stickability on a skin surface and such a good sense of fitness for a skin surface as not to cause a sticking sensation under affixation to the skin surface but also allows reductions in sweaty and itchy sensations under affixation to the skin surface, and further can relieve a physical irritation to the skin surface when peeled away from the skin surface.

### BRIEF DESCRIPTION OF THE DRAWINGS

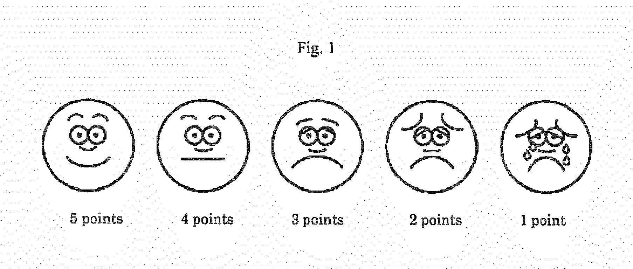

[Fig. 1] Figure 1 includes patterns illustrating "Face Pain Scale" used for evaluation of pains caused at the time when each of the adhesive skin patch materials prepared in Examples 2 to 5 and Comparative Examples 2 to 5 was peeled away from a skin surface.

### DETAILED DESCRIPTION OF THE INVENTION

In the invention, the polyurethane elastomer film functioning as a base film to support a pressure-sensitive adhesive layer described later is required to have a thickness of I µm to 9 µm, and the thickness thereof is preferably from 3 µm to 9 µm.

When the thickness of polyurethane elastomer film is below 1 µm, there is a potential for an adhesive skin patch material incorporating such polyurethane elastomer film to be broken at the time when the adhesive skin patch material is put to use or peeled away from the skin surface after it has been used. On the other hand, when the thickness of polyurethane elastomer film exceeds 9 µm, there may be cases where an adhesive skin patch material incorporating such polyurethane elastomer film cannot follow elastic movement of a skin surface to cause a sense of incongruity, such as a stiff sensation, under affixation of the adhesive skin patch material to the skin surface.

Additionally, a reason for using polyurethane elastomer film as a base film is that this elastomer film is superior in elasticity and moisture permeability, and therefore the film not only can follow movements of a flexion portion of a body but also can prevent the skin from getting sweaty and suffering rash.

### (Pressure-Sensitive Adhesive Layer)

The pressure-sensitive adhesive layer contains an acrylic-based copolymer because such a copolymer allows easy adjustment to adhesion of the pressure-sensitive adhesive layer to skin, reduction in irritation to the skin when the pressure-sensitive adhesive layer is brought into contact with skin, and so on.

The acrylic-based copolymer has no particular restrictions, but it is appropriate to use an acrylic-based copolymer produced from a monomer mixture including e.g. a (meth)acrylic alkyl ester monomer and an alkoxy group-containing ethylenic unsaturated monomer.

A (meth)acrylic alkyl ester monomer becomes a main ingredient for imparting good stickability on skin to the pressure-sensitive adhesive layer, and it is especially effective to use the monomer whose alkyl group is at least 6 in carbon number, preferably a long-chain alkyl group containing 6 to 18 carbon atoms. Additionally, (meth)acrylic alkyl ester monomers are advantageous in that they are relatively weak in stimulation to skin and resist causing reduction in adhesion even by long-duration use.

Examples of such a (meth)acrylic acid alkyl ester monomer include butyl ester, propyl ester, octyl ester, nonyl ester, decyl ester, dodecyl ester and lauryl ester of acrylic acid or methacrylic acid. These esters may be used alone, or as combinations of two or more thereof. By the way, it is needless to say that alkyl ester chains in these monomers may be either straight chains or branched chains.

The alkoxy group-containing ethylenic unsaturated monomer is an ingredient for imparting water-vapor permeability, or the so-called moisture permeability, to an acrylic-based copolymer. The alkoxy group-containing ethylenic unsaturated monomer having such an action is not limited to particular ones, but it is appropriate to use e.g. an alkoxyalkyl acrylate having a 1-4C alkoxy group, with examples including methoxypolyethylene glycol acrylate, ethoxydiethylene glycol acrylate, butoxydiethylene glycol acrylate, methoxyethyl acrylate, ethoxyethyl acrylate and butoxyethyl acrylate.

Furthermore, as the acrylic-based copolymer in the invention, it is also appropriate to use an acrylic-based copolymer produced from a monomer mixture further containing a carboxyl group-containing ethylenic unsaturated monomer. By containing a carboxyl group-containing ethylenic unsaturated monomer, the copolymer obtained increases in cohesive force, and therefore this additional monomer becomes an important monomer in point of adjustment to properties of the pressure-sensitive adhesive layer. Representative examples of such a monomer include acrylic acid, itaconic acid, crotonic acid, fumaric acid and maleic acid (anhydride). Of these acids, acrylic acid is preferred over the others from the viewpoints of copolymerizability and handling ease.

One which is suitable as the acrylic-based copolymer which is a constituent of the pressure-sensitive adhesive is a copolymerization product of the foregoing two kinds of monomers, namely a (meth)acrylic alkyl ester monomer and an alkoxy group-containing ethylenic unsaturated monomer, and one which is more suitable as the acrylic-based copolymer is a copolymerization product of the foregoing three kinds of monomers, namely a (meth)acrylic alkyl ester monomer, an alkoxy group-containing ethylenic unsaturated monomer and a carboxyl group-containing ethylenic unsaturated monomer.

In producing the acrylic-based copolymer, monomers for making various modifications, e.g. for imparting hydrophilicity, may be copolymerized as appropriate in addition to the above-recited monomers. Examples of a monomer for modification use include styrene, vinyl acetate and N-vinyl-2-pyrrolidone.

It is appropriate that the content of a (meth)acrylic alkyl ester monomer in the acrylic-based copolymer be from 40 mass% to 80 mass%, preferably from 50 mass% to 75 mass%. When the content of a (meth)acrylic alkyl ester monomer is in the above-specified range, the pressure-sensitive adhesive layer formed shows good stickability on skin and, at the time of peeling from the skin, it does not cause a phenomenon that an adhesive residue is left on the skin, and has excellent peelability.

On the other hand, it is appropriate that the content of an alkoxy group-containing ethylenic unsaturated monomer be from 10 mass% to 60 mass%, preferably from 20 mass% to 60 mass%, far preferably from 25 mass% to 50 mass%. When the content of an alkoxy group-containing ethylenic unsaturated monomer is in the above-specified range, the resulting copolymer can impart excellent moisture permeability to the pressure-sensitive adhesive layer.

In the case of additionally incorporating a carboxyl group-containing ethylenic unsaturated monomer, it is appropriate that the alkoxy group-containing ethylenic unsaturated monomer content be adjusted to fall within a range of 10 mass% to 50 mass%, preferably 20 mass% to 45 mass%.

It is appropriate that the content of a carboxyl group-containing ethylenic unsaturated monomer be adjusted to fall within a range of 1 mass% to 10 mass%, preferably 3 mass% to 8 mass%. When the content of a carboxyl group-containing ethylenic unsaturated monomer is in the above-specified range, the resulting copolymer can impart excellent cohesive force to the pressure-sensitive adhesive layer, and the adhesive residue phenomenon and the like can be controlled, and besides stimulation to skin can be reduced.

It is appropriate that, in the portion dissolved in a solvent for molecular-weight measurement, the acrylic-based copolymer be adjusted to have a weight-average molecular weight in a range of 0.5 million to 2.5 million (500,000 to 2,500,000), preferably about 0.6 million to about 2.0 million (600,000 to 2,000,000).

By the way, the weight-average molecular weight as mentioned above and the distribution of molecular weights are values determined using gel permeation chromatography (GPC), and a sample for measurement is dissolved in tetrahydrofuran, and the sample's soluble portion having passed through a 0.45-µmφ membrane filter is subjected to GPC measurement and those values are calculated in terms of polystyrene.

The pressure-sensitive adhesive layer contains an ingredient in a liquid or pasty state at room temperature (25°C). The ingredient in a liquid or pasty state at room temperature is incorporated because of its actions of imparting flexibility to the pressure-sensitive adhesive layer and reducing stimulation to skin when the pressure-sensitive adhesive layer is brought into contact with the skin.

Such an ingredient in a liquid or pasty state at room temperature has no particular restrictions so long as it is compatible with acrylic-based copolymers. Examples of an ingredient defined above include esters produced from alkyl alcohols and acids such as phthalic acid, maleic acid, adipic acid and various fatty acids including stearic acid, and esters produced from acids and polyhydric alcohols such as ethylene glycol and glycerin. To be more specific, examples of a monohydric alcohol ester include dibutyl phthalate, di-2-ethylhexyl phthalate, dibutyl adipate, di-2-ethylhexyl sebacate, dibutyl maleate, ethyl myristate, isopropyl myristate, isopropyl palmitate, butyl stearate, isopropyl isostearate, hexyl laurate, cetyl lactate, myristyl lactate, diethyl phthalate, octyldodecyl myristate, octyldodecyl oleate, hexyldecyl dimethyloctanoate, cetyl 2-ethylhexanoate, isocetyl 2-ethylhexanoate, stearyl 2-ethylhexanoate and dioctyl succinate. And examples of a dihydric or higher alcohol ester include propylene glycol dicaprylate, propylene glycol dicaprate, propylene glycol diisostearate, glyceryl monocaprylate, glyceryl tricaprylate, glyceryl tri-2-ethylhexanoate, glyceryl tricaprate, glyceryl trilaurate, glyceryl triisostearate, glyceryl trioleate and trimethylolpropane tri-2-ethylhexanoate. These esters can be used alone, or any two or more thereof can be used in combination. Such an ingredient as to be in a liquid or pasty state at room temperature can be chosen as appropriate according to other factors in the adhesive skin patch material, but from the viewpoint of compatibility with acrylic-based copolymers, the ingredient used suitably is a carboxylic acid ester, more preferably a glyceryl fatty acid ester, still more preferably a glycerin ester of a saturated fatty acid.

Examples of a fatty acid as mentioned above include saturated fatty acids such as caprylic acid, capric acid and 2-ethylhexanoic acid, and these acids can be used alone or as combinations of two or more thereof. Because such a saturated fatty acid has no unsaturated double bond, the resultant pressure-sensitive adhesive, or equivalently the resultant pressure-sensitive adhesive layer, can be prevented from suffering oxidative deterioration.

When such caprylic acid and like acid as recited above are used as saturated fatty acids, esters of various saturated fatty acids and alcohols are obtained, with examples including glyceryl tricaprylate, glyceryl tricaprate and glyceryl tri-2-ethylhexanoate. Of these carboxylic acid esters, glyceryl tricaprylate is preferred over the others in point of compatibility with acrylic-based copolymers.

As to the content ratio between an acrylic-based copolymer and an ingredient in a liquid or pasty state at room temperature, such as a carboxylic acid ester as recited above, it is appropriate that the ingredient content be, say, from 20 to 100 parts by mass with respect to 100 parts by mass of an acrylic-based copolymer. When the ingredient has its content in the foregoing range, it can impart sufficient flexibility to a pressure-sensitive adhesive layer, and therefore the resultant pressure-sensitive adhesive layer not only can have sufficient stickability on skin, but also can ensure reduced physical stimulation to skin and satisfactory peelability at the occasion of peeling from the skin.

For the purpose of imparting a moderate cohesive force to the pressure-sensitive adhesive layer as a constituent member of the present adhesive skin patch material, it is appropriate that the pressure-sensitive adhesive layer undergo crosslinking treatment. As the crosslinking treatment, there are physical crosslinking by the irradiation with ionizing radiation, such as electron beams, γ rays or X rays, and chemical crosslinking by the use of a crosslinking agent, but from the viewpoints of treatability and good reproducibility in crosslinking, it is preferred that the pressure-sensitive adhesive layer be subjected to chemical crosslinking treatment using a crosslinking agent. For example, a crosslinking agent is added to a pressure-sensitive adhesive composition in which materials to form a pressure-sensitive adhesive layer are mixed and the pressure-sensitive adhesive layer formed from the composition is subjected to heat treatment to undergo crosslinking. Examples of such a crosslinking agent include crosslinking agents usable in giving crosslinking treatment, such as isocyanate-type crosslinking agents, peroxide-type crosslinking agents and metal chelate-type crosslinking agents.
Although the pressure-sensitive adhesive layer having undergone the crosslinking treatment as mentioned above keeps a balance between moderate stickability on skin and cohesive force in the pressure-sensitive adhesive layer through the control of a crosslinking degree, the molecular weight and molecular-weight distribution of the acrylic-based copolymer also exert an influence on adjustment to such a balance.
The pressure-sensitive adhesive layer thickness 20 to 35 µm. By doing so, the pressure-sensitive adhesive layer can impart a moderate stickiness to the skin surface,
For the purpose of preventing the pressure-sensitive adhesive layer surface from being contaminated, it is appropriate that the pressure-sensitive adhesive layer surface of the adhesive skin patch material be covered with a release liner until there comes a time when it is put to use. As such a release liner, those generally used for skin and pressure-sensitive adhesive tapes for affixation to skin can be used. To be more specific, a release liner usable herein is one which is formed by coating the surface of wood free paper, glassine paper, parchment paper or so on with a release agent having releasability, such as a silicone resin or a fluorocarbon resin, one which is formed by anchor-coating wood free paper with a resin, or one which is formed by coating the surface of a polyethylene laminate with a release agent having releasability, such as a silicone resin or a fluorocarbon resin.

Additionally, a supporting film, though not even shown graphically, may be included in the adhesive skin patch material in a state of being laminated releasably to one side of the base film opposite to the side on which the pressure-sensitive adhesive layer is formed.

Suitable examples of a material for the supporting film include plastic films (e.g. polyethylene film, polypropylene film, polyester film and laminated composites thereof) and paper (e.g. wood free paper, craft paper). Of these materials, plastic film having transparency can exert outstanding usefulness on the occasion of fixing a medical instrument such as catheter because it allows affixation of an adhesive skin patch material under observation of a sticking region via the adhesive skin patch material. In order to laminate releasably the supporting film to the rear side of the base film, it is possible to use a known method, such inflation molding, extrusion laminate molding, lamination molding or casting.

The suitable thickness of a supporting film, depending on a raw material of the film, is generally on the order of 15 to 200 µm, preferably on the order of 20 to 100 µm.

In addition, though not even shown graphically, the adhesive skin patch material can be wound into a roll body in a state of being laminated to the supporting film as mentioned above.

Next, an example of a method for manufacturing the present adhesive skin patch material is illustrated below, but this example should not be construed as limiting the scope of the invention in any way.

First a monomer mixture including e.g. a (meth)acrylic alkyl ester monomer and an alkoxyl group-containing ethylenic unsaturated monomer, and optionally a carboxyl group-containing ethylenic unsaturated monomer, is subjected to copolymerization reaction according to general radical polymerization in an organic solvent to prepare an acrylic-based copolymer solution. Then, into the acrylic-based copolymer solution is mixed an ingredient in a liquid or pasty state at room temperature, such as a carboxylic acid ester, in an amount of 20 to 100 parts by mass with respect to 100 parts by mass of solids in the acrylic-based copolymer solution (acrylic-based copolymer), and further thereto are added a crosslinking agent and so on as required. Thus a pressure-sensitive adhesive composition solution is prepared.

Next the pressure-sensitive adhesive solution thus obtained is applied to all over or part of the release-treated surface of a release liner. When it is partially applied, the pressure-sensitive adhesive solution is preferably applied in the form of e.g. dots or streaks. In the case of applying the solution in the form of streaks, it is possible to adopt e.g. linear coating or undulatory coating, but any coating technique may be used so long as spaces functioning as gas vent passages, namely spaces between streaks, are secured. The undulatory coating is preferable because the time-dependent change in the cross-sectional area of spaces between streaks is little under sticking on skin. After the pressure-sensitive adhesive solution applied to the release liner is dried, thereon is stuck a supporting film-laminated polyurethane elastomer film to give an adhesive skin patch material.

### Examples

The invention will now be illustrated in more detail by reference to examples, but these examples should not be construed as limiting the scope of the invention. Additionally, all parts and percentages in the following examples are parts by mass and mass%, respectively.

### (Example 1)

Into an acrylic-based copolymer solution produced by inducing copolymerization reaction in a monomer mixture constituted of 65 parts of isononyl acrylate, 30 parts of 2-methoxyethyl acrylate and 5 parts of acrylic acid were mixed glyceryl tricaprylate and a trifunctional isocyanate compound as crosslinking agent in amounts of 60 parts and 0.04 parts, respectively, with respect to 100 parts of the acrylic-based copolymer, thereby preparing a homogeneous pressure-sensitive adhesive solution.

Then, the thus prepared solution was applied to the release-treated surface of a release liner, which had undergone on one side thereof release treatment with a silicone, so as to have a thickness of 26 µm after drying, and then dried to form a pressure-sensitive adhesive layer. This pressure-sensitive adhesive layer was stuck on the polyurethane elastomer film side of a laminate film which had been formed by laminating releasably a 40 µm-thick polypropylene film (supporting film) to an 8 µm-thick polyurethane elastomer film, and thereby an adhesive skin patch material was produced as Example 1.

### (Comparative Example 1)

An adhesive skin patch material as Comparative Example 1 was produced in the same manner as in Example 1, except that the 8 µm-thick polyurethane elastomer film was changed to a 30 µm-thick polyurethane elastomer film and the thickness of the pressure-sensitive adhesive layer after drying was changed to 30 µm.

### (Moisture Permeability)

10 mL of purified water was put into a 38 mm-diameter glass vessel, and the mouth of the glass vessel was covered with an adhesive skin patch material prepared by peeling the release liner and the supporting film away from each of the adhesive skin patch materials produced in Example 1 and Comparative Example 1, and was sealed by winding a pressure-sensitive adhesive tape round the periphery of the mouth portion of the glass vessel. After weight measurement was made on such a sealed glass vessel, the vessel was stored for 24 hours under a condition that the temperature and the humidity be adjusted to 40°C and 30% RH, respectively. After 24-hour storage, the weight measurement was also made on the thus stored glass vessel, and an amount of water vapor having passed through the adhesive skin patch material was determined from a difference in weight of the glass vessel between before and after the storage. From a value of this difference, a moisture permeation amount (moisture permeability) per m² of test specimen was worked out (a mean value in the case of n=3).

### (Sensory Evaluation under Stuck Condition)

Square test pieces with dimensions of 50 mm wide by 50 mm long were cut out of each of the adhesive skin patch materials produced in Example 1 and Comparative Example 1, and after the release liners were peeled away, the pressure-sensitive adhesive layer sides of the test pieces were stuck on the humeral regions of 5 volunteers, respectively. Then, the supporting films were peeled away, and the resultant test pieces were left as they were for 3 hours at room temperature. In the skin region at this time, the sensation of stiffness, the unstuck condition in the end portion of each adhesive skin patch material and the degree of sweatiness were each rated on a 1-to-3 scale. In addition, the degree of pain caused at the time of peeling of each adhesive skin patch material was also rated on a 1-to-3 scale.

**Table 1**

| | Moisture Permeability [g/m² 24h] |
|---|---|
| Example 1 | 1,588 |
| Comparative Example 1 | 1,014 |

**Table 2**

| | Sensation of Stiffness | Unstuck Condition | Degree of Pain | Degree of Sweatiness |
|---|---|---|---|---|
| Example 1 | 3 | 3 | 3 | 3 |
| Comparative Example 1 | 1 | 3 | 3 | 2 |

### (Sensation of Stiffness)

3: There is no sensation of stiffness.
2: There is a slight sensation of stiffness.
1: There is an obvious sensation of stiffness.

### (Unstuck Condition)

3: There is no unstuck area.
2: There is a slightly unstuck area in the end portion.
1: Obviously unstuck area is present in more than half of the affixation region.

### (Degree of Pain)

3: No pain is caused.
2: A slight pain is caused.
1: An appreciable pain is caused.

### (Degree of Sweatiness)

3: No sweat accumulates in the affixation region.
2: Sweat accumulates a little in the affixation region.
1: Sweat accumulates appreciably in the affixation. region.

Test results obtained have been shown in Table 1 and Table 2.

The adhesive skin patch material as Example 1 caused neither accumulation of sweat nor formation of unstuck area in the end portion under affixation to the skin, or equivalently, it showed satisfactory stickability on the skin. In addition, the adhesive skin patch material as Example 1 showed the property of following well movements of the skin, hardly gave a sensation of stiffness to the skin, and created a good sense of fitness for the skin without causing perception of its affixation. Further, despite its good stickability on the skin, the adhesive skin patch material as Example 1 left no adhesive residue, and gave no physical stimulation (pain) to the skin at the time of peeling.

On the other hand, the adhesive skin patch material as Comparative Example 1 gave a sensation of stiffness to the skin under its affixation, and created a perception of its affixation. In addition, the adhesive skin patch material as Comparative Example 1 accumulated sweat in the region of its affixation in some cases.

### (Example 2)

Into an acrylic-based copolymer solution obtained by inducing copolymerization reaction in a monomer mixture constituted of 65 parts of isononyl acrylate, 30 parts of 2-methoxyethyl acrylate and 5 parts of acrylic acid were mixed glyceryl tricaprylate and a trifunctional isocyanate compound as crosslinking agent in amounts of 60 parts and 0.13 parts, respectively, with respect to 100 parts of the acrylic-based copolymer, thereby preparing a homogeneous pressure-sensitive adhesive solution.

Then, the thus prepared solution was applied to the release-treated surface of a release liner, which had undergone on one side thereof release treatment with a silicone, so as to have a thickness of 23 µm after drying, and then dried to form a pressure-sensitive adhesive layer. This pressure-sensitive adhesive layer was stuck on the polyurethane elastomer film side of a laminate film, which had been formed by laminating releasably a 40 µm-thick polypropylene film (supporting film) to an 8 µm-thick polyurethane elastomer film (base film), and thereby an adhesive skin patch material was produced as Example 2.

### (Examples 3 to 5 and Comparative Examples 2 and 3)

Each of adhesive skin patch materials as Examples 3 to 5 and Comparative Examples 2 and 3 was produced in the same manner as in Example 2, except that the thickness of the polyurethane elastomer film and the thickness of the pressure-sensitive adhesive layer after drying were changed to values as shown in Table 3, respectively.

### (Comparative Example 4)

An adhesive skin patch material as Comparative Example 4 was produced in the same manner as in Example 2, except that the homogeneous pressure-sensitive adhesive solution was prepared without incorporating the glyceryl tricaprylate and the trifunctional isocyanate compound as crosslinking agent.

### (Comparative Example 5)

An adhesive skin patch material as Comparative Example 5 was produced in the same manner as in Example 2, except that the 8 µm-thick polyurethane elastomer film was changed to a 6 µm-thick polyethylene terephthalate film.

A summary of the adhesive skin patch materials as Examples 2 to 5 and Comparative Examples 2 to 5 is shown in Table 3.

**Table 3**

| | Base Film | Thickness of [µm] | Thickness of Pressure-Sensitive Adhesive Layer [µm] | Remarks |
|---|---|---|---|---|
| Example 2 | PU | 8 | 23 | |
| Example 3 | PU | 5 | 23 | |
| Example 4 | PU | 2 | 23 | |
| Example 5 | PU | 8 | 16 | |
| Comparative Example 2 | PU | 13 | 23 | |
| Comparative Example 3 | PU | 30 | 23 | |
| Comparative Example 4 | PU | 8 | 23 | Neither liquid ingredient nor crosslinking agent is incorporated |
| Comparative Example 5 | PET | 6 | 23 | |

| | | | | |
|---|---|---|---|---|
| PU: Polyurethane elastomer film PET: Polyethylene terephthalate film | | | | |

### (Modulus)

As to the adhesive skin patch materials as Examples 2 to 5 and Comparative Examples 2 to 5, each adhesive skin patch material was cut out into a rectangular piece whose dimensions were 10 mm wide by 100 mm long, and after its release liner was peeled away, the rectangular piece was mounted so that the distance between chucks of a tensile strength tester was adjusted to 40 mm. Then, after its supporting film was peeled away, the rectangular piece was pulled at a speed of 300 mm/min, and loads (N/10 mm) at the points in time when the values of percentage elongation reached 10%, 30% and 100%, respectively, were read (mean values in the case of n=3).

### (Moisture Permeability)

Moisture permeability measurements were made on the adhesive skin patch materials as Examples 2 to 5 and Comparative Examples 2 to 5, respectively, in accordance with the same method as used for the adhesive skin patch materials as Example 1 and Comparative Example 1.

### (Stickiness)

As to the adhesive skin patch materials as Examples 2 to 5 and Comparative Examples 2 to 5, each adhesive skin patch material was cut out into a rectangular piece whose dimensions were 19 mm wide by about 70 mm long, and after its release liner was peeled away, the rectangular piece was wound round a region covering from the first articulations to the second articulations of fingers of a test subject, and then its supporting film was peeled away. The resultant adhesive skin patch material was pressed for 30 seconds with the palm of the other hand of the test subject, and allowed to remain in the wound state for 5 minutes. Thereafter, the adhesive skin patch material was peeled away, and the degree of stickiness on the fingers at the time of this peeling (a mean value in the case of n=5) was rated in a 1-to-5 scale.
5: No stickiness is created and the condition remains the same as before peeling,
4: An oily ingredient is perceived slightly to be left.
3: A slight stickiness is perceived.
2: A slight adhesive residue is seen by visual check.
1: An appreciable adhesive residue is seen by visual check.

### (Pain at the Time of Peeling)

As to the adhesive skin patch materials as Examples 2 to 5 and Comparative Examples 2 to 5, each adhesive skin patch material was cut out into a rectangular piece whose dimensions were 19 mm wide by about 70 mm long, and after its release liner was peeled away, the rectangular piece was wound round a region covering from the first articulations to the second articulations of fingers of a test subject, and then its supporting film was peeled away. The resultant adhesive skin patch material was pressed for 30 seconds with the palm of the other hand of the test subject, and allowed to remain in the wound state for 5 minutes. Thereafter, the adhesive skin patch material was peeled away, and the degree of pain caused by this peeling (a mean value in the case of n=5) was rated in a 1-to-5 scale according to "Face Pain Scale" shown in Fig. 1.

### <Score in Rating>

5 points: No pain is perceived.
4 points: A slight pain is perceived
3 points: A pain on a bearable level is perceived.
2 points: An appreciable pain is perceived.
1 point: An unbearable pain is perceived.

**Table 4**

| | Modulus [N/10 mm] | | | Moisture Permeability [g/m² 24h] | Stickiness | Pain at Peeling |
|---|---|---|---|---|---|---|
| | 10% | 30% | 100% | | | |
| Example 2 | 0.10 | 0.27 | 0.52 | 1,265 | 4.8 | 5,0 |
| Example 3 | 0.08 | 0.18 | 0.33 | 1,434 | 5.0 | 4.8 |
| Example 4 | 0.04 | 0.09 | 0.18 | 1,533 | 5.0 | 5.0 |
| Example 5 | 0.07 | 0.24 | 0.49 | 1,422 | 4.4 | 5.0 |
| Comparative Example 2 | 0.19 | 0.57 | 1.19 | 1,210 | 4.2 | 5.0 |
| Comparative Example 3 | 0.23 | 0.65 | 1.29 | 995 | 3.8 | 4.8 |
| Comparative Example 4 | 0.20 | 0.33 | 0.60 | 1,158 | 3.8 | 3.5 |
| Comparative Example 5 | 7.48 | Broken | Broken | 94 | 4.0 | 4.0 |

Test results are shown in Table 4.

The adhesive skin patch materials as Examples 2 to 5 were low in modulus, and hence it followed that they gave such a good sense of fitness for the skin as to make the test subjects forget the affixation thereof, and besides they showed high moisture permeability, thereby hardly inducing sweatiness under affixation to the skin. In addition, the adhesive skin patch materials as Examples 2 to 5 showed good results in the tests for the stickiness on the skin after peeling and the pain at the time of peeling, and more specifically they had skin-friendly properties including the property of hardly causing an itchy sensation under affixation to the skin and the property of reducing physical stimulation to the skin at the time of peeling.

On the other hand, the adhesive skin patch materials as Comparative Examples 2 and 3 were higher in modulus than the adhesive skin patch materials as Examples 2 to 5, and thereby showed a tendency to give an inferior sense of fitness under affixation to the skin, and besides, they were somewhat lower in moisture permeability, and thereby showed a tendency to create easily stickiness on the skin. The adhesive skin patch material as Comparative Example 5 was obviously high in modulus, gave a bad sense of fitness under affixation to the skin, and was low in moisture permeability to result in easy creation of stickiness on the skin.

Because the difference between the adhesive patch material as Example 2 and the adhesive patch material as Comparative Example 4 consisted in the presence or absence of the liquid or pasty ingredient, it is thought that the presence of the liquid or pasty ingredient in the adhesive skin patch material as Example 2 made it possible to produce not only an effect of alleviating the pain at the time of peeling but also an unexpected effect of increasing the moisture permeability to contribute to a high score on the stickiness rating.

Up to this point the invention has been illustrated in detail on the basis of the foregoing Examples, but the invention should not be construed as being limited to those Examples, and various changes and modifications can be made without departing from the spirit and scope of the invention.

While the invention has been described in detail and with reference to specific embodiments thereof, it will be apparent to one skilled in the art that various changes and modifications can be made therein without departing from the scope thereof.

This application is based on Japanese patent application No. 2016-156971 filed August 9, 2016, the entire contents thereof being hereby incorporated by reference.

## Claims

1. An adhesive skin patch material, which comprises a polyurethane elastomer film, a pressure-sensitive adhesive layer provided on one side of the polyurethane elastomer film, and a release liner releasably laminated so as to cover the sticky surface of the pressure-sensitive adhesive layer,
wherein the polyurethane elastomer film has a thickness of 1 µm to 9 µm,
wherein the pressure-sensitive adhesive layer has a thickness of 20 to 35 µm, and
wherein the pressure-sensitive adhesive layer contains an acrylic-based copolymer and an ingredient in a liquid or pasty state at room temperature.

2. The adhesive skin patch material of claim 1, wherein the ingredient in a liquid or pasty state at room temperature is a carboxylic acid ester.

3. The adhesive skin patch material of claim 2, wherein the carboxylic acid ester is a glycerin ester of a saturated fatty acid.

4. The adhesive skin patch material of claim 3, wherein the saturated fatty acid is at least one selected from the group consisting of caprylic acid, capric acid and 2-ethylhexanoic acid.

5. The adhesive skin patch material of any one of claims 2 to 4, wherein the carboxylic acid ester is at least one selected from the group consisting of glyceryl tricaprylate, glyceryl tricaprate and glyceryl tri-2-ethylhexanoate.

6. The adhesive skin patch material of any one of claims 1 to 5, wherein the acrylic-based copolymer contains an acrylic-based copolymer produced from a monomer mixture comprising a (meth)acrylic alkyl ester monomer and an alkoxy group-containing ethylenic unsaturated monomer.

7. The adhesive skin patch material of any one of claims 1 to 6, wherein the ingredient in a liquid or pasty state at room temperature is incorporated in an amount of 20 to 100 parts by mass with respect to 100 parts by mass of the acrylic-based copolymer in the pressure-sensitive adhesive layer.

8. The adhesive skin patch material of any one of claims 1 to 7, further comprising a supporting film which is releasably laminated so as to cover the other surface of the polyurethane elastomer film.

9. A roll body of an adhesive skin patch material, which is formed by winding the adhesive skin patch material of claim 8 into a roll body.

## Patentansprüche

1. Klebendes Hautpflastermaterial, umfassend einen Polyurethan-Elastomer-Film, eine druckempfindliche Klebeschicht, die auf einer Seite des Polyurethan-Elastomer-Filmes vorgesehen ist, und eine Abziehlage, die abziehbar derart auflaminiert ist, dass sie die haftende Oberfläche der druckempfindlichen Klebeschicht bedeckt,
wobei der Polyurethan-Elastomer-Film eine Dicke von 1 µm bis 9 µm aufweist, wobei die druckempfindliche Klebeschicht eine Dicke von 20 bis 35 µm aufweist, und
wobei die druckempfindliche Klebeschicht ein acrylbasiertes Copolymer und eine Komponente in flüssigem oder pastösem Zustand bei Raumtemperatur enthält.

2. Klebendes Hautpflastermaterial nach Anspruch 1, wobei die Komponente in flüssigem oder pastösem Zustand bei Raumtemperatur ein Carboxylsäureester ist.

3. Klebendes Hautpflastermaterial nach Anspruch 2, wobei der Carboxylsäureester ein Glycerinester einer gesättigten Fettsäure ist.

4. Klebendes Hautpflastermaterial nach Anspruch 3, wobei die gesättigte Fettsäure wenigstens eine ist, die aus einer Gruppe ausgewählt ist, die aus Caprylsäure, Caprinsäure und 2-Ethylhexansäure ausgewählt ist.

5. Klebendes Hautpflastermaterial nach einem der Ansprüche 2 bis 4, wobei der Carboxylsäureester wenigstens einer ist, der aus einer Gruppe ausgewählt ist, die aus Glyceryltricaprylat, Glyceryltricaprat und Glyceryl-tri-2-ethylhexanoat ausgewählt ist.

6. Klebendes Hautpflastermaterial nach einem der Ansprüche 1 bis 5, wobei das acrylbasierte Copolymer ein acrylbasiertes Copolymer enthält, das aus einem Monomer-Gemisch hergestellt ist, das ein (Meth)acrylalkylester-Monomer und ein alkoxygruppenhaltiges ethylenisches ungesättigtes Monomer umfasst.

7. Klebendes Hautpflastermaterial nach einem der Ansprüche 1 bis 6, wobei die Komponente in flüssigem oder pastösem Zustand bei Raumtemperatur in einer Menge von 20 bis 100 Massenteile bezogen auf 100 Massenteile des acrylbasierten Copolymers in die druckempfindliche Klebeschicht eingebracht ist.

8. Klebendes Hautpflastermaterial nach einem der Ansprüche 1 bis 7, des Weiteren umfassend einen unterstützenden Film, der abziehbar derart auflaminiert ist, dass er die andere Oberfläche des Polyurethan-Elastomer-Filmes bedeckt.

9. Rollenkörper für ein klebendes Hautpflastermaterial, der dadurch gebildet ist, dass das klebende Hautpflastermaterial von Anspruch 8 auf einen Rollenkörper gewickelt wird.

## Revendications

1. Matériau de timbre cutané adhésif, qui comprend un film élastomère de polyuréthane, une couche adhésive sensible à la pression disposée sur une face du film élastomère de polyuréthane et un revêtement antiadhésif stratifié de manière amovible afin de recouvrir la surface collante de la couche adhésive sensible à la pression,
dans lequel le film élastomère de polyuréthane a une épaisseur de 1 µm à 9 µm,
dans lequel la couche adhésive sensible à la pression a une épaisseur de 20 à 35 µm, et
dans lequel la couche adhésive sensible à la pression contient un copolymère à base d'acrylique et un ingrédient à l'état liquide ou pâteux à température ambiante.

2. Matériau de timbre cutané adhésif selon la revendication 1, dans lequel l'ingrédient à l'état liquide ou pâteux à température ambiante est un ester d'acide carboxylique.

3. Matériau de timbre cutané adhésif selon la revendication 2, dans lequel l'ester d'acide carboxylique est un ester de glycérine d'un acide gras saturé.

4. Matériau de timbre cutané adhésif selon la revendication 3, dans lequel l'acide gras saturé est au moins l'un choisi dans le groupe constitué par l'acide caprylique, l'acide caprique et l'acide 2-éthylhexanoïque.

5. Matériau de timbre cutané adhésif selon l'une quelconque des revendications 2 à 4, dans lequel l'ester d'acide carboxylique est au moins l'un choisi dans le groupe constitué par le tricaprylate de glycéryle, le tricaprate de glycéryle et le tri-2-éthylhexanoate de glycéryle.

6. Matériau de timbre cutané adhésif selon l'une quelconque des revendications 1 à 5, dans lequel le copolymère à base d'acrylique contient un copolymère à base d'acrylique produit à partir d'un mélange de monomères comprenant un monomère ester d'alkyle (méth)acrylique et un monomère à insaturation éthylénique contenant un groupe alcoxy.

7. Matériau de timbre cutané adhésif selon l'une quelconque des revendications 1 à 6, dans lequel l'ingrédient à l'état liquide ou pâteux à température ambiante est incorporé en une quantité de 20 à 100 parties en masse par rapport à 100 parties en masse du copolymère à base d'acrylique dans la couche adhésive sensible à la pression.

8. Matériau de timbre cutané adhésif selon l'une quelconque des revendications 1 à 7, comprenant en outre un film de support qui est stratifié de manière amovible afin de recouvrir l'autre surface du film élastomère de polyuréthane.

9. Corps de rouleau d'un matériau de timbre cutané adhésif, qui est formé en enroulant le matériau de timbre cutané adhésif de la revendication 8 sous forme de corps de rouleau.
